# EUROPEAN PATENT SPECIFICATION

(11) **EP 4 360 630 B1**
(45) Date of publication and mention of the grant of the patent: **03.06.2026**
(21) Application number: 22872943.0
(22) Date of filing: 21.09.2022
(51) Int. Cl.: A61K 31/196, A61K 9/70, A61P 29/00

(54) **METHOD FOR INHIBITING GENERATION OF DICLOFENAC INDOLINONES**
VERFAHREN ZUR HEMMUNG DER BILDUNG VON DICLOFENAC-INDOLINONEN
PROCÉDÉ D'INHIBITION DE PRODUCTION D'INDOLINONES DE DICLOFÉNAC

(30) Priority: 27.09.2021 JP 2021156757
(43) Date of publication of application: 01.05.2024
(73) Proprietor: Hisamitsu Pharmaceutical Co., Inc., Tosu-shi, Saga 841-0017 (JP)
(72) Inventor: FUJITA, Naoko, Tsukuba-shi, Ibaraki 305-0856 (JP); IWAMOTO, Nao, Tsukuba-shi, Ibaraki 305-0856 (JP); HIROSE, Kazuyuki, Tosu-shi, Saga 841-0017 (JP); UCHIDA, Naoyuki, Tsukuba-shi, Ibaraki 305-0856 (JP)
(74) Representative: Grünecker Patent- und Rechtsanwälte PartG mbB
(86) International application number: PCT/JP2022/035204
(87) International publication number: WO 2023/048192

(56) References cited:
- WO-A1-00/24386
- WO-A1-2013/191128
- JP-A- 2003 520 191
- JP-A- 2011 121 980
- JP-A- 2019 055 928
- US-A1- 2015 202 171
- US-B1- 8 852 628

## Description

### Technical Field

The present invention relates to a method for suppressing generation of diclofenac indolinone.

### Background Art

Patches comprising diclofenac, one of non-steroidal anti-inflammatory analgesics, have been known to have efficacies such as analgesia and anti-inflammatory action for diseases as well as symptoms, such as a lumbago, scapulohumeral periarthritis, a cervicobrachial syndrome, tendon and tendon sheath inflammation, peritendinitis, humeral epicondylitis (tennis elbow and the like), muscle pain (muscular·myofascial back pain and the like), and tumidity and pain after traumatic injury, and efficacies of analgesia for various types of cancer, and the patches have been extensively investigated. For example, Patent Literature 1 describes a patch containing diclofenac or a pharmaceutically acceptable salt thereof, citric acid and dimethyl sulfoxide, wherein a mass ratio of diclofenac or a pharmaceutically acceptable salt thereof to citric acid and a mass ratio of diclofenac or a pharmaceutically acceptable salt thereof to dimethyl sulfoxide are in prescribed ranges. Patent Literature 2 also describes an active substance-containing adhesive patch medicine consisting of an impermeable auxiliary film, diclofenac or a pharmaceutically acceptable salt thereof, a matrix-forming polymer selected from a styrene-isoprene-styrene block copolymer and an ethylene-vinyl acetate copolymer, a tackifier consisting of an aliphatic hydrocarbon resin and a thermoplastic terpene resin as well as one or more solvents selected from the group consisting of oleic acid and a derivative thereof, a fatty acid alkyl ester and a N-alkyl-pyrrolidone, and a releasable protective film.
US 2015/202171 relates to a patch comprising a support layer and an adhesive layer, wherein the adhesive layer comprises diclofenac sodium, dimethyl sulfoxide, and citric acid.
WO 00/24386 relates to methods of preparing pressure sensitive adhesive matrix patch devices for transdermal drug delivery, more particularly to the preparation of pressure sensitive adhesive matrix patches by dissolving hydrophilic salts of hydrophobic drugs that are crystalline at room temperature in aqueous dispersions of hydrophobic pressure sensitive adhesive polymers.
JP2019-055928 relates to a patch containing a diclofenac salt, an organic acid and a metal oxide, and more specifically, to a diclofenac salt, an organic acid as a main drug solubilizer, and a metal oxide as a diclofenac salt stabilizer.

### Citation List

### Patent Literature

[Patent Literature 1] WO2013/191128
[Patent Literature 2] JP2004-508397

### Summary of Invention

### Technical Problem

Most of the diclofenac-containing patches that have been studied so far are those containing a pharmaceutically acceptable salt of diclofenac (specifically, diclofenac sodium). In patches containing the pharmaceutically acceptable salt of diclofenac, diclofenac indolinone are known to be generated, which represent a type of degradation products of diclofenac. In order to provide a highly stable patch, suppression of generation of diclofenac indolinone is of importance. Therefore, an object of the present invention is to provide a method for suppressing generation of diclofenac indolinone.

### Solution to Problem

The present inventors investigated patches containing diclofenac free acid and have found that a patch containing the prescribed amount of diclofenac free acid can suppress generation of diclofenac indolinone more than a patch containing diclofenac sodium and thus have completed the present invention.

Namely the present invention provides a method for suppressing generation of diclofenac indolinone in an adhesive layer, in a patch comprising a backing layer and an adhesive layer laminated on the backing layer, comprising a step of mixing diclofenac free acid and an adhesive base to obtain an adhesive composition; and a step of forming the adhesive composition to obtain the patch, wherein a content of the diclofenac free acid is 0.5% by mass to 8% by mass based on a total mass of the adhesive layer.

### Advantageous Effects of Invention

According to the present invention, generation of diclofenac indolinone (particularly generation of diclofenac indolinone upon storage) in a patch, can be suppressed.

### Description of Embodiments

The present invention will be described in detail below by demonstrating embodiments of the present invention.

The patch comprises a backing layer and an adhesive layer laminated on the backing layer. The adhesive layer contains diclofenac free acid and an adhesive base, and the content of the diclofenac free acid is 0.5% by mass to 8% by mass based on the total mass of the adhesive layer.

The backing layer supports the adhesive layer. It is preferable that the backing layer is a single-layer body or a laminated body of a cloth-like fiber (woven fabric, non-woven fabric, or knitted fabric), or a non-porous or porous film (sheet). It is preferable that a material of the backing layer is one or more materials selected from a polyester (such as polyethylene terephthalate, polyethylene isophthalate, polypropylene terephthalate, polypropylene isophthalate, polybutylene terephthalate, polyethylene naphthalate), a polyolefin (a polymer or a copolymer of a vinyl monomer such as ethylene, propylene, vinyl acetate, or acrylonitrile), a polyamide (such as nylon or silk), a polyurethane, and cellulose (such as cotton or linen). Cloth (woven fabric, non-woven fabric, or knitted fabric) may be coated with a rubber composition. The rubber composition comprises a rubber-based adhesive base. The rubber-based adhesive base includes, for example, a polyisoprene, a polyisobutylene, a polybutadiene, a styrene-butadiene-styrene block copolymer, a styrene-isoprene-styrene block copolymer, styrene-butadiene rubber, styrene-isoprene rubber, or combinations thereof. The rubber composition may also comprise a tackifier resin. The tackifier resin includes, for example, an alicyclic saturated hydrocarbon resin, a hydrogenated rosin glycerin ester, a terpene-based resin, or combinations thereof. The rubber composition may also further comprise additives such as a plasticizer and a filler. The thickness of the backing layer is, for example, 0.1 mm to 2 mm. The basis weight of the backing layer is, for example, 30 g/m² to 200 g/m². In the present specification, the thickness and the basis weight of the backing layer are measured in accordance with the standard of JIS L 1906:2000.

It is preferable that moisture permeability of the backing layer is 400 g/m²·24 hours or more, it is more preferable that the moisture permeability is 1,000 g/m²·24 hours or more, and it is further preferable that the moisture permeability is 4,000 g/m²/24 hours or more. When a backing layer having such high moisture permeability is used, dimethyl sulfoxide gradually volatilizes from a patch applied to the skin. Therefore, adhesion of the patch is improved, the patch is unlikely to peel off even it is applied for a long time. The upper limit value of the moisture permeability may be 20,000 g/m²·24 hours. When the moisture permeability of the backing layer is within such a range of, dimethyl sulfoxide more easily volatilizes from the adhesive layer, therefore the backing layer is more effective for improving the adhesion of the patch. The moisture permeability of the backing layer is moisture permeability at 40°C which is defined in accordance with the JIS Z0208:1976 (moisture permeability test method (cup method) of the moisture-proof packaging materials).

In a case where the backing layer has a cloth shape, it is preferable that a 50% modulus (JIS L 1018:1999) of the backing layer in either a longitudinal direction (material flow direction) or a lateral direction (material width direction) is also 1 N/50 mm to 12 N/50 mm. In a case where the 50% modulus is 12N/50mm or less, the amount of stress applied to the patch due to expansion and contraction of the skin is smaller, and therefore the adhesion to the skin is favorable.

In the case where the backing layer is a film, it is preferable that the material thereof is one such as a polyurethane having highly moisture permeable (highly dimethyl sulfoxide permeable). Since a film made of polyurethane is excellent in stretchability, the polyurethane is thereby preferable from the viewpoint of enhancing adhesion of the patch to the skin and stretch followability.

It is preferable that the backing layer is, for example, a non-woven fabric or a film made of polyurethane, a knitted fabric, a non-woven fabric or a film made of polyethylene terephthalate, a polyester cloth coated with a rubber composition, or combinations thereof. More specifically, it is more preferable that the backing layer is a laminated body of a film made of polyurethane and a non-woven fabric made of polyurethane, a knitted fabric or a non-woven fabric made of polyethylene terephthalate, or a polyester cloth coated with a rubber composition.

The adhesive layer is formed of an adhesive composition obtained by mixing diclofenac free acid, an adhesive base, and an optional component as described below. It is preferable that the overall adhesive mass of the adhesive layer is 10 g/m² to 1,000 g/m², it is more preferable that the overall adhesive mass is 30 g/m² to 300 g/m², and it is further preferable that the overall adhesive mass is 100 g/m² to 250 g/m², from the viewpoint of appropriately adhering a patch to the skin.

Diclofenac free acid is a compound also called 2-[(2,6-dichlorophenyl)amino]phenylacetic acid. The content of diclofenac free acid is 0.5% by mass to 8% by mass, and may be 1% by mass to 7% by mass, 1% by mass to 5% by mass or 1% by mass to 4.65% by mass, based on the total mass of the adhesive layer.

The adhesive base is to impart adhesion to the adhesive layer. Examples thereof include a rubber-based adhesive base, an acrylic-based adhesive base, and a silicone-based adhesive base. It is preferable that the adhesive base is one or more types selected from the group consisting of the rubber-based adhesive base, acrylic-based adhesive base, and silicone-based adhesive base. The adhesive base that does not comprise water (nonaqueous adhesive base), is preferable. The adhesive base may be any of the rubber-based adhesive base, the acrylic-based adhesive base, or the silicone-based adhesive base, or combinations thereof. The total content of the adhesive base can be 10% by mass to 90% by mass and may be 20% by mass to 90% by mass, based on the total mass of the adhesive layer.

Examples of the rubber-based adhesive base include natural rubber, a polyisobutylene, an alkyl vinyl ether (co)polymer, a polyisoprene, a polybutadiene, a styrene-butadiene copolymer, a styrene-isoprene copolymer, a styrene-isoprene-styrene block copolymer, and a styrene-butadiene-styrene block copolymer. The rubber-based adhesive base may be used singly among them, or in combination of two or more. Among them, it is preferable that the rubber-based adhesive base according to the present embodiment is at least one or more selected from the group consisting of the styrene-isoprene-styrene block copolymer and polyisobutylene from the viewpoint of having a likelihood of exhibiting more sufficient adhesive strength in an adhesive layer, and it is more preferable that the adhesive layer is the styrene-isoprene-styrene block copolymer, or both the styrene-isoprene-styrene block copolymer and polyisobutylene.

Specific examples of the rubber-based adhesive base include QUINTAC^{®} 3570C (trade name, manufactured by ZEON Corporation), SIS5002, SIS5229, SIS5505, SIS5505P (trade names, manufactured by JSR Corporation), and SIBSTAR^{®} T102 (trade name, manufactured by Kaneka Corporation). Examples of the polyisobutylene also include so-called butyl rubber (isobutylene-isoprene rubber), and specific examples thereof include OPPANOL^{®} N50, N80, N100, N150, B11, B12, B50, B80, B100, B120, B150, B220 (trade names, manufactured by BASF Corporation), JSR^{®} Butyl 065, 268, 365 (trade names, manufactured by JSR Corporation), X-BUTYL^{®} RB 100, 101-3, 301, 402 (trade names, manufactured by ARLANXEO Inc.), and EXXON^{®} Butyl 065, 065S, 068, 068S, 268, 268S, 365, 365S (trade names, manufactured by Exxon Mobile Corporation).

The content of the rubber-based adhesive base can be 10% by mass to 90% by mass and may be 15% by mass to 60% by mass, 15% by mass to 40% by mass, or 15% by mass to 30% by mass, based on the total mass of an adhesive layer. In a case in which a mixture of a styrene-isoprene-styrene block copolymer and a polyisobutylene is used as the rubber-based adhesive base, it is preferable that a mass ratio of the both is 4:1 to 1:4, 3:1 to 1:1, or 3:1 to 2:1.

The acrylic-based adhesive base is a component that imparts adhesion to an adhesive layer and is, for example, a (co)polymer of one or two or more of (meth)acrylic acid alkyl esters. Examples of the (meth)acrylic acid alkyl ester include butyl (meth)acrylate, isobutyl (meth)acrylate, hexyl (meth)acrylate, octyl (meth)acrylate, 2-ethylhexyl (meth)acrylate, and decyl (meth)acrylate. The term "(meth)acrylic acid" used herein refers to either or both acrylic acid and methacrylic acid, and analogous expressions are also similarly defined.

The acrylic-based adhesive base may be a copolymer formed of an (meth)acrylic acid alkyl ester (main monomer) and a comonomer. Examples of the main monomer include methyl (meth)acrylate, ethyl (meth)acrylate, butyl (meth)acrylate, hexyl (meth)acrylate, heptyl (meth)acrylate, octyl (meth)acrylate, and 2-ethylhexyl (meth)acrylate, and these may be used singly, or in combination of two or more. The comonomer may be a component that can copolymerize with a (meth)acrylic acid alkyl ester. Examples of the comonomer include a (meth)acrylic acid hydroxyalkyl ester, ethylene, propylene, styrene, vinyl acetate, N-vinyl pyrrolidone, (meth)acrylic acid, and (meth)acrylic acid amide. The comonomers may be used singly, or in combination of two or more.

Specific examples of the acrylic-based adhesive base include an acrylic acid-octyl acrylate copolymer, a 2-ethylhexyl acrylate-vinyl pyrrolidone copolymer solution, an acrylic ester-vinyl acetate copolymer, a 2-ethylhexyl acrylate-2-ethylhexyl methacrylate-dodecyl methacrylate copolymer, a methyl acrylate-2-ethylhexyl acrylate copolymer resin emulsion, and an acrylic polymer contained in an acrylic resin alkanolamine solution. Specific examples of such acrylic-based adhesive bases include DURO-TAK^{®} series (manufactured by Henkel Corporation), such as DURO-TAK^{®} 387-2510, DURO-TAK^{®} 87-2510, DURO-TAK^{®} 387-2287, DURO-TAK^{®} 87-2287, DURO-TAK^{®} 87-4287, DURO-TAK^{®} 387-2516, DURO-TAK^{®} 87-2516, DURO-TAK^{®} 87-2074, DURO-TAK^{®} 87-900A, DURO-TAK^{®} 87-901A, DURO-TAK^{®} 87-9301, and DURO -TAK^{®} 87-4098; GELVA^{®} series (manufactured by Henkel Corporation), such as GELVA^{®} GMS 788, GELVA^{®} GMS 3083, and GELVA^{®} GMS 3253; MAS^{®} series (manufactured by CosMED Pharmaceutical Co., Ltd.), such as MAS^{®} 811 (trade name) and MAS^{®} 683 (trade name); EUDRAGIT^{®} series (manufactured by Evonik Industries AG), NICASOL^{®} series (manufactured by Nippon Carbide Industry Co., Inc.), and ULTRAZOL^{®} series (manufactured by AICA Kogyo Company, Limited).

The content of the acrylic-based adhesive base can be 10% by mass to 90% by mass and may be 20% by mass to 90% by mass, based on the total mass of an adhesive layer.

The silicone-based adhesive base is a compound having an organopolysiloxane skeleton. Examples of the silicone-based adhesive base include a dimethylpolysiloxane, a polymethylvinylsiloxane, and a polymethylphenylsiloxane. Specific examples of the silicone-based adhesive base include MD series (manufactured by DuPont Toray Specialty Materials Co., Ltd.), such as MD7-4502 Silicone Adhesive and MD7-4602 Silicone Adhesive; BIO-PSA^{®} series (manufactured by DuPont Toray Specialty Materials Co., Ltd.), such as BIO-PSA^{®} 7-4301 Silicone Adhesive, BIO-PSA^{®} 7-4302 Silicone Adhesive, BIO-PSA^{®} 7-4201 Silicone Adhesive, BIO-PSA^{®} 7-4202 Silicone Adhesive, BIO-PSA^{®} 7-4101 Silicone Adhesive, BIO-PSA^{®} 7-4102 Silicone Adhesive, BIO-PSA^{®} 7-4601 Silicone Adhesive, BIO-PSA^{®} 7-4602 Silicone Adhesive, BIO-PSA^{®} 7-4501 Silicone Adhesive, BIO-PSA^{®} 7-4502 Silicone Adhesive, BIO-PSA^{®} 7-4401 Silicone Adhesive, and BIO-PSA^{®} 7-4402 Silicone Adhesive; DOW CORNING^{®} 7-9800A, DOW CORNING^{®} 7-9800B, DOW CORNING^{®} 7-9700A, and DOW CORNING^{®} 7-9700B.

The content of the silicone-based adhesive base can be 10% by mass to 90% by mass and may be 20% by mass to 90% by mass, based on the total mass of the adhesive layer.

The adhesive layer may further contain dimethyl sulfoxide for the purpose of dissolving diclofenac free acid to improve skin permeability of the adhesive layer. It is preferable that the content of dimethyl sulfoxide is 1% by mass to 20% by mass, 1% by mass to 7% by mass, 2% by mass to 10% by mass, 3% by mass to 10% by mass, or 4% by mass to 8% by mass, based on the total mass of the adhesive layer.

It is preferable that a ratio of diclofenac free acid to dimethyl sulfoxide is 1:0.3 to 1:14, 1:0.4 to 1:7, 1:0.6 to 1:3, or 1:0.7 to 1:3 in terms of improving skin permeability of the diclofenac free acid and preventing precipitation of diclofenac crystal.

The adhesive layer may arbitrarily further contain other additive. Examples of the other additive include a tackifier resin, a plasticizer, an absorption enhancer, a solubilizer, a stabilizer, a filler, and a fragrance.

The tackifier resin is a component that adjusts adhesion of the adhesive layer. Examples of the tackifier resin include a petroleum-based resin, a terpene-based resin, a rosin-based resin, a phenol-based resin, and a xylene-based resin. Examples of the petroleum-based resin include an alicyclic petroleum resin (such as an alicyclic saturated hydrocarbon resin), an aliphatic petroleum resin (such as an aliphatic hydrocarbon resin), and an aromatic petroleum resin and more specific examples thereof include ARKON^{®} P-70, ARKON^{®} P-85, ARKON^{®} P-90, ARKON^{®} P-100, ARKON^{®} P-115, ARKON^{®} P-125, ARKON^{®} M-70, ARKON^{®} M-85, ARKON^{®} M-90, ARKON^{®} M-100, ARKON^{®} M-115, and ARKON^{®} M-125 (the above all are trade names and manufactured by Arakawa Chemical Industries, Ltd.); and ESCOREZ^{®} 8000 (trade name, manufactured by Esso Petrochemical Company, Limited). Examples of the terpene-based resin include pinene polymers (such as an α-pinene polymer and a β-pinene polymer), terpene polymers, dipentene polymers, terpene-phenol polymers, aromatic modified terpene polymers, and pinene-phenol copolymers, and specific examples thereof include YS Resin (such as YS Resin PXN (1150N, 300N), YS Resin PX1000, YS Resin TO125, or YS Resin TO105), CLEARON P105, CLEARON M115, CLEARON K100 (the above all are trade names and manufactured by Yasuhara Chemical Co Ltd.), and Tamanol 901 (trade name, manufactured by Arakawa Chemical Industries, Ltd.). Examples of the rosin-based resin include a hydrogenated rosin glycerin ester, an ultra-pale rosin, an ultra-pale rosin ester, and an acid modified ultra-pale rosin, and more specific examples thereof include Pine Crystal (such as KE-311, PE-590, KE-359, or KE-100) (trade names, manufactured by Arakawa Chemical Industries, Ltd.). The tackifier resins may be used singly among them, or in combination of two or more. Among these, it is preferable that the tackifier resin is the alicyclic saturated hydrocarbon resin, terpene-based resin, hydrogenated rosin glycerin ester, or combinations thereof, and it is more preferable that the tackifier resin is the alicyclic saturated hydrocarbon resin, hydrogenated rosin glycerin ester, or combinations thereof. When an adhesive layer contains the tackifier resin, the content of the tackifier resin can be 15% by mass to 80% by massand may be 30% by mass to 65% by mass, based on the total mass of the adhesive layer. In a case in which the alicyclic saturated hydrocarbon resin and the hydrogenated rosin glycerin ester are used as the tackifier resins, it is preferable that the mass ratio of the two is 4:1 to 1 :4, 4: 1 to 2: 3, or 3: 1 to 2:1.

Examples of the plasticizer include paraffin oil (such as liquid paraffin), squalane, squalene, vegetable oils (such as olive oil, camellia oil, castor oil, tall oil, peanut oil, spearmint oil, eucalyptus oil, jojoba oil, camphor white oil, sunflower oil, and orange oil), fats and oils (such as dibutyl phthalate and dioctyl phthalate), and liquid rubber (such as liquid polybutene and liquid isoprene rubber). A preferred plasticizer is liquid paraffin. The plasticizers may be used singly, or in combination of two or more. When an adhesive layer contains the plasticizer, the content of the plasticizer is, for example, 3% by mass to 50% by mass, 5% by mass to 30% by mass, or 7% by mass to 20% by mass, based on the total mass of the adhesive layer.

The absorption enhancer may be any compounds conventionally known to have a percutaneous absorption-enhancing effect. Examples of the absorption enhancer include organic acids and salts thereof (for example, aliphatic carboxylic acids having 6 to 20 carbon atoms (hereinafter also referred to as "fatty acids") and salts thereof, and cinnamic acid and salts thereof), organic acid esters (for example, fatty acid esters and cinnamic acid esters), organic acid amides (for example, fatty acid amides), aliphatic alcohols, polyhydric alcohols, and ethers (for example, aliphatic ethers and polyoxyethylene alkyl ethers). These absorption enhancers may have an unsaturated bond and may be cyclic, linear or branched chemical structures. The absorption enhancers may also be a monoterpene compound, a sesquiterpene compound, and vegetable oil (for example, olive oil). These absorption enhancers may be used singly, or in combination of two or more.

Examples of such organic acids include aliphatic (mono-, di- or tri-) carboxylic acids (such as acetic acid, propionic acid, isobutyric acid, caproic acid, caprylic acid, fatty acid, lactic acid, maleic acid, pyruvic acid, oxalic acid, succinic acid, and tartaric acid), aromatic carboxylic acids (such as phthalic acid, salicylic acid, benzoic acid, and acetylsalicylic acid), cinnamic acid, alkanesulfonic acids (such as methanesulfonic acid, ethanesulfonic acid, propanesulfonic acid, and butanesulfonic acid), alkylsulfonic acid derivatives (such as a polyoxyethylene alkyl ether sulfonic acid and N-2-hydroxyethylpiperazine-N'-2-ethanesulfonic acid), and cholic acid derivatives (such as dehydrocholic acid). These organic acids may be alkali metal salts such as sodium salts. Among these, the aliphatic carboxylic acid, and the aromatic carboxylic acid or salts thereof are preferable, and acetic acid or sodium acetate is particularly preferable. Examples of the fatty acid include lauric acid, myristic acid, palmitic acid, stearic acid, isostearic acid, oleic acid, linoleic acid, and linolenic acid.

In another embodiment, the adhesive layer is free of either citric acid (including anhydrous citric acid) or a salt thereof. In a patch comprising the adhesive layer free of citric acid or a salt thereof, generation of diclofenac indolinone is more suppressed.

Examples of the organic acid ester include ethyl acetate, propyl acetate, cetyl lactate, lauryl lactate, methyl salicylate, ethylene glycol salicylate, methyl cinnamate, and fatty acid ester. Examples of the fatty acid ester include methyl laurate, hexyl laurate, isopropyl myristate, myristyl myristate, octyldodecyl myristate, isopropyl palmitate, and cetyl palmitate. The fatty acid ester may be a glycerin fatty acid ester, a propylene glycol fatty acid ester, a sorbitan fatty acid ester, a sorbitan fatty acid ester-polyethylene glycol ether, a polyethylene glycol fatty acid ester, a sucrose fatty acid ester, or polyoxyethylene cured castor oil. Specific examples of the fatty acid ester include glycerin monocaprylate, glycerin monocaprate, glycerin monolaurate, glycerin monooleate, sorbitan monolaurate, sucrose monolaurate, Polysorbate 20, propylene glycol monolaurate, polyethylene glycol monolaurate, polyethylene glycol monostearate, Span 40, Span 60, Span 80, Span 120 (trade names, manufactured by Croda Japan KK), TWEEN^{®} 20, TWEEN^{®} 21, TWEEN^{®} 40, TWEEN^{®} 60, TWEEN^{®} 80, and NIKKOL^{®} HCO-60 (trade names, manufactured by Nikko Chemicals Co., Ltd.).

Examples of the organic acid amide include fatty acid amides (for example, lauric acid diethanolamide), hexahydro-1-dodecyl-2H-azepin-2-one (also referred to as Azone) and derivatives thereof, and pyrothiodecane.

The aliphatic alcohol refers to an aliphatic alcohol having 6 to 20 carbon atoms. Examples of the aliphatic alcohol include lauryl alcohol, myristyl alcohol, oleyl alcohol, isostearyl alcohol, and cetyl alcohol. Examples of polyhydric alcohols include propylene glycol.

The aliphatic ether refers to an ether having an aliphatic group having 6 to 20 carbon atoms (for example, an alkyl group and an alkenyl group). Examples of a polyoxyethylene alkyl ether include a polyoxyethylene lauryl ether.

Examples of the monoterpene compound include geraniol, thymol, terpineol, l-menthol, borneol, d-limonene, isoborneol, nerol, and dl-camphor. Mentha oil may be used as the monoterpene compound.

When an adhesive layer contains the absorption enhancer, the content of the absorption enhancer can be 2% by mass to 40% by mass based on the total mass of the adhesive layer.

The solubilizer is a component that facilitates diclofenac free acid to dissolve into the adhesive composition. Examples of the solubilizer include fatty acid alkyl esters (for example, isopropyl myristate and isopropyl palmitate), fatty acid polyhydric alcohol esters (for example, propylene glycol monolaurate, glyceryl monolaurate, glyceryl monooleate, and sorbitan monolaurate), fatty acid amides (for example, lauric acid diethanolamide), aliphatic alcohols (for example, octyldodecanol, isostearyl alcohol, and oleyl alcohol), polyhydric alcohols (for example, a propylene glycol, a dipropylene glycol, and a polyethylene glycol), pyrrolidone derivatives (for example, N-methyl-2-pyrrolidone), and amines (for example, triethanolamine, diethanolamine, monoethanolamine, diisopropanolamine, trometamol, and meglumine). The solubilizer may be used singly, or in combination of two or more. When an adhesive layer contains the solubilizer, the content of the solubilizer can be 2% by mass to 40% by mass based on the total mass of the adhesive layer.

The stabilizer may be those capable of suppressing generation of free radicals resulting from the action of a ray such as an ultraviolet ray, heat, or active chemical species, and progress of a chain reaction of the free radicals. Examples of the stabilizer include tocopherol and ester derivatives thereof, ascorbic acid and ester derivatives thereof, 2,6-dibutylhydroxytoluene, butyl hydroxyanisole, and 2-mercaptobenzimidazole. The stabilizers may be used singly, or in combination of two or more. When an adhesive layer contains the stabilizer, the content of the stabilizer can be 0.05% by mass to 3% by mass and may be 0.05% by mass to 1% by mass, 0.05% by mass to 0.25% by mass, or 0.1% by mass to 0.25% by mass, based on the total mass of the adhesive layer.

Examples of the filler include powders of metal compounds (such as aluminum oxide, aluminum hydroxide, zinc oxide, titanium oxide, and calcium carbonate), ceramics (such as talc, clay, kaolin, silica, hydroxyapatite, synthetic aluminum silicate, and magnesium metasilicate aluminate) or organic compounds (such as cellulose powder and stearate), or short fibers of resins including these materials. The fillers may be used singly, or in combination of two or more. When an adhesive layer contains the filler, the content of the filler can be 0.1% by mass to 20% by mass based on the total mass of the adhesive layer.

The patch may further comprise a release liner. The release liner is laminated on an opposite surface of the backing layer to the adhesive layer. The patch comprising the release liner tends to allow adhesion of dust to the adhesive layer to be reduced upon storage. It is preferable that a surface of the release liner contacting the adhesive layer undergoes release treatment by using silicone, or a fluorinated polyolefin.

As a material for the release liner, liners commonly known to those skilled in the art can be used. Examples of the release liner include paper; polyesters such as a polyethylene terephthalate and a polyethylene naphthalate; polyolefins such as a polyethylene and a polypropylene; films such as a polyvinyl chloride, a polyvinylidene chloride, nylon, and aluminum. The release liner may be a laminate film of high-quality paper and a polyolefin. It is preferable that the material for the release liner is a film made of polypropylene or polyethylene terephthalate.

The method for manufacturing a patch comprises a step of mixing diclofenac free acid and an adhesive base to obtain an adhesive composition; and a step of forming the adhesive composition to obtain the patch. The patch can be more specifically produced, for example, by the following method. First, respective components constituting an adhesive layer are mixed in a predetermined ratio to obtain a homogeneously dissolved material (adhesive composition). Next, the adhesive composition is spread over a releasable film (release liner) or backing layer in the predetermined adhesive mass to form an adhesive layer. Subsequently, the adhesive layer is pressed and adhered onto the backing layer or releasable film so that the adhesive layer is sandwiched between the releasable film and the backing layer. Finally, the resultant can be cut into a desired shape to obtain a patch. In this case, the release liner is removed when the patch is applied.

The method for suppressing generation of diclofenac indolinone in an adhesive layer, according to one embodiment of the present invention, comprises a step of mixing diclofenac free acid and an adhesive base to obtain an adhesive composition; and a step of forming the adhesive composition to obtain the patch. More specifically, the method can be implemented by the same method as the method for manufacturing the aforementioned patch.

An area of the patch may be 5 to 200 cm², and may be 30 to 100 cm². A shape and dimensions of the patch may be, for example, a rectangle with a short side of 3 to 14 cm and a long side of 7 to 20 cm, or a circle with a diameter of 1 to 10 cm.

### Examples

### Experiment 1: Evaluation of generation rate of diclofenac indolinone in patch using rubber-based adhesive base

### 1. Preparation of patch

In accordance with Table 1 below, each component was mixed to obtain an adhesive composition. The obtained adhesive composition was spread over a release liner (film made of polyethylene terephthalate subjected to release treatment) so that the adhesive mass per unit area was 214 g/m², to form an adhesive layer. A backing layer (woven fabric made of polyethylene terephthalate) was laminated on the surface of the obtained adhesive layer opposite to the release liner to obtain a patch in which a backing layer/an adhesive layer/a release liner were laminated in this order.

### 2. Measurement of content of diclofenac indolinone

The content of diclofenac indolinone was measured by the following procedure by using a patch immediately after manufacture and a patch after having stored in an aluminum packaging bag at 60°C for one week after manufacture.

First, an adhesive layer of the patch was taken out and immersed in 2 mL of acetone to extract an organic substance.

Next, 6 mL of diluted solution (0.1% phosphoric acid aqueous solution/methanol = 60/40 (v/v)) was added thereto to make them 8 mL in total, an insoluble substance was filtered off, and then by using high-performance liquid chromatography under the following analysis conditions, a chromatogram was obtained, in which peaks of diclofenac free acid and diclofenac indolinone were separated. The content of diclofenac indolinone was calculated from an area under the peak curve corresponding to the diclofenac indolinone, with the theoretical amount of diclofenac free acid being 100.

### <Analysis conditions>

Column: YMC-Pack Pro C8
Mobile phase: 0.1% phosphoric acid aqueous solution/methanol = 60/40 (v/v)
Measurement wavelength: 275 nm
Flow rate: 1 mL/min
Sample injection volume: 30 µL
Column temperature: 40°C

**[Table 1]**

| | Comp. Ex. 1 | Ex. 1 | Ex. 2 | Ex. 3 | Ex. 4 | Comp. Ex. 2 |
|---|---|---|---|---|---|---|
| Diclofenac free acid | - | 1.00 | 3.00 | 4.65 | 7.00 | 10.00 |
| Diclofenac sodium | 5.00 | - | - | - | - | - |
| Styrene-isoprene-styrene block copolymer | 19.15 | 20.89 | 20.32 | 19.85 | 19.18 | 18.33 |
| Polyisobutylene | 8.21 | 8.96 | 8.71 | 8.51 | 8.24 | 7.86 |
| Aliphatic saturated hydrocarbon resin | 27.00 | 29.45 | 28.65 | 27.99 | 27.05 | 25.84 |
| Hydrogenated rosin glycerin ester | 10.00 | 10.00 | 10.00 | 10.00 | 10.00 | 10.00 |
| Liquid paraffin | 12.53 | 13.66 | 13.30 | 12.99 | 12.55 | 11.99 |
| Oleic acid | 5.00 | 5.00 | 5.00 | 5.00 | 5.00 | 5.00 |
| Dimethyl sulfoxide | 7.00 | 7.00 | 7.00 | 7.00 | 7.00 | 7.00 |
| Anhydrous citric acid | 2.11 | - | - | - | - | - |
| Others | 4.00 | 4.04 | 4.02 | 4.01 | 4.0 | 3.98 |
| Total | 100 | 100 | 100 | 100 | 100 | 100 |
| Generation rate of indolinone immediately after production (%) | 0.84 | 0.13 | 0.15 | 0.21 | 0.22 | 0.42 |
| Generation rate of indolinone after one week at 60°C (%) | 2.18 | 0.33 | 0.55 | 0.65 | 1.36 | 2.57 |

The patches of Examples 1 to 4 containing diclofenac free acid were confirmed to be able to suppress generation of diclofenac indolinone more than the patch of Comparative Example 1 containing diclofenac sodium. In the case of the patch of Comparative Example 2, in which the content of diclofenac free acid was 10% by mass based on the total mass of the adhesive layer, the patch was confirmed not to be able to suppress the generation of diclofenac indolinone.

### Experiment 2: Evaluation of generation rate of diclofenac indolinone in patch using acrylic-based adhesive base

### 1. Preparation of patch

In accordance with Table 2 below, each component was mixed to obtain an adhesive composition. The obtained adhesive composition was spread over a release liner (film made of polyethylene terephthalate subjected to release treatment) so that the adhesive mass per unit area was 120 g/m², and a solvent was removed by drying to form an adhesive layer. A backing layer (film made of polyethylene terephthalate) was laminated on the surface of the obtained adhesive layer opposite to the release liner to obtain a patch in which a backing layer/an adhesive layer/a release liner were laminated in this order.

### 2. Measurement of content of diclofenac indolinone

The content of diclofenac indolinone was measured in the same manner as in Experiment 1.

**[Table 2]**

| | Comparative Example 3 | Example 5 |
|---|---|---|
| Diclofenac free acid | - | 4.65 |
| Diclofenac sodium | 5.00 | - |
| Acrylic-based adhesive base (MAS811B) | 77.89 | 80.35 |
| Oleic acid | 5.00 | 5.00 |
| Dimethyl sulfoxide | 7.00 | 7.00 |
| Anhydrous citric acid | 2.11 | - |
| Others | 3.00 | 3.00 |
| Total | 100 | 100 |
| Generation rate of indolinone immediately after production (%) | 0.09 | 0.06 |
| Generation rate of indolinone after one week at 60°C (%) | 2.05 | 0.84 |

Also in the case of using the acrylic-based adhesive base, the patch of Example 5 containing diclofenac free acid was confirmed to be able to suppress the generation of diclofenac indolinone more than the patch of Comparative Example 3 containing diclofenac sodium.

### Experiment 3: Evaluation of generation rate of diclofenac indolinone in patch using silicone-based adhesive base

### 1. Preparation of patch

A patch was manufactured in the same manner as in Experiment 2, in accordance with the composition in Table 3 below. However, in this Example, the adhesive composition was spread over so that the adhesive mass per unit area was 160 g/m².

### 2. Measurement of content of diclofenac indolinone

The content of diclofenac indolinone was measured in the same manner as in Experiment 1.

**[Table 3]**

| | Comparative Example 4 | Example 6 |
|---|---|---|
| Diclofenac free acid | - | 4.65 |
| Diclofenac sodium | 5.00 | - |
| Silicone-based adhesive base (BIO-PSA 7-4202) | 77.89 | 80.35 |
| Oleic acid | 5.00 | 5.00 |
| Dimethyl sulfoxide | 7.00 | 7.00 |
| Anhydrous citric acid | 2.11 | - |
| Others | 3.00 | 3.00 |
| Total | 100 | 100 |
| Generation rate of indolinone immediately after production (%) | 0.21 | 0.06 |
| Generation rate of indolinone after one week at 60°C (%) | 5.32 | 1.23 |

Also in the case of using the silicone-based adhesive base, the patch of Example 6 containing diclofenac free acid was confirmed to be able to suppress the generation of diclofenac indolinone more than the patch of Comparative Example 4 containing diclofenac sodium.

### Experiment 4: Evaluation of generation rate of diclofenac indolinone in patch free of polyisobutylene

### 1. Preparation of patch

A patch was manufactured in the same manner as in Experiment 1, in accordance with the composition in Table 4 below.

### 2. Measurement of diclofenac indolinone content

The content of diclofenac indolinone was measured in the same manner as in Experiment 1.

**[Table 4]**

| | Example 7 |
|---|---|
| Diclofenac free acid | 4.65 |
| Styrene-isoprene-styrene block copolymer | 22.76 |
| Polyisobutylene | - |
| Aliphatic saturated hydrocarbon resin | 32.10 |
| Hydrogenated rosin glycerin ester | 10.00 |
| Liquid paraffin | 14.89 |
| Oleic acid | 5.00 |
| Dimethyl sulfoxide | 7.00 |
| Others | 3.60 |
| Total | 100 |
| Generation rate of indolinone immediately after production (%) | 0.17 |
| Generation rate of indolinone after one week at 60°C (%) | 1.03 |

Even in the case of using the patch free of isobutylene, it was confirmed that the patch containing diclofenac free acid, of Example 7 could suppress the generation of diclofenac indolinone more than the patch containing diclofenac sodium, of Comparative Example 1. The generation rate of the diclofenac indolinone of the patch of Example 7 was also confirmed to be approximately equal compared to those of the patches in Examples 1 to 4.

### Experiment 5: Evaluation of generation rate of diclofenac indolinone in patch using acrylic-based adhesive base or silicone-based adhesive base

### 1. Preparation of patch

Patches of Examples 8 and 9 were manufactured in the same manner as in Experiment 2, in accordance with the composition in Table 5 below. Patches of Examples 10 and 11 were also manufactured in the same manner as in Experiment 3, in accordance with the composition in Table 5 below.

### 2. Measurement of diclofenac indolinone content

The content of diclofenac indolinone was measured in the same manner as in Experiment 1.

**[Table 5]**

| | Example 8 | Example 9 | Example 10 | Example 11 |
|---|---|---|---|---|
| Diclofenac free acid | 1.00 | 7.00 | 1.00 | 4.65 |
| Acrylic-based adhesive base (MAS811B) | 84.00 | 78.00 | - | - |
| Silicone-based adhesive base (BIO-PSA 7-4202) | - | - | 90.00 | - |
| Silicone-based adhesive base (BIO-PSA 7-4502) | - | - | - | 80.35 |
| Oleic acid | 5.00 | 5.00 | 5.00 | 5.00 |
| Dimethyl sulfoxide | 7.00 | 7.00 | 1.00 | 7.00 |
| Others | 3.00 | 3.00 | 3.00 | 3.00 |
| Total | 100 | 100 | 100 | 100 |
| Generation rate of indolinone immediately after production (%) | 0.00 | 0.04 | 0.00 | 0.18 |
| Generation rate of indolinone after one week at 60°C (%) | 0.29 | 1.34 | 0.57 | 0.48 |

Also in the case of using the acrylic-based adhesive base or silicone-based adhesive base, each of the patches of Examples 8 to 10, in which the content of diclofenac free acid based on the total mass of the adhesive layer was varied within the range of 0.5% by mass to 8% by mass, was confirmed to be able to more suppress the generation of diclofenac indolinone compared to the patch of Comparative Example 3 or 4 containing diclofenac sodium. The patch of Example 11, which used the silicone-based adhesive base different from the patch of Example 6, was also confirmed to be capable of more suppressing the generation of diclofenac indolinone than the patch containing diclofenac sodium, of Comparative Example 4.

## Claims

1. A method for suppressing generation of diclofenac indolinone in an adhesive layer, in a patch comprising a backing layer and the adhesive layer laminated on the backing layer, comprising:
a step of mixing diclofenac free acid and an adhesive base to obtain an adhesive composition; and
a step of forming the adhesive composition to obtain the patch,
wherein a content of the diclofenac free acid is 0.5% by mass to 8% by mass based on a total mass of the adhesive layer.

## Patentansprüche

1. Verfahren zur Unterdrückung der Bildung von Diclofenac-Indolinon in einer Klebstoffschicht in einem Pflaster, umfassend eine Trägerschicht und die auf die Trägerschicht laminierte Klebstoffschicht, umfassend:
einen Schritt des Vermischens von freier Diclofenac-Säure und einer Klebstoffbasis, um eine Klebstoffzusammensetzung zu erhalten; und
einen Schritt des Formens der Klebstoffzusammensetzung, um das Pflaster zu erhalten,
wobei der Gehalt an freier Diclofenac-Säure 0,5 Massen-% bis 8 Massen-%, bezogen auf die Gesamtmasse der Klebstoffschicht, beträgt.

## Revendications

1. Procédé de suppression de la génération de diclofénac indolinone dans une couche adhésive, dans un timbre comprenant une couche dorsale et la couche adhésive stratifiée sur la couche dorsale, comprenant :
une étape de mélange d'acide libre de diclofénac et d'une base adhésive pour obtenir une composition adhésive ; et
une étape de mise en forme de la composition adhésive pour obtenir le timbre,
dans lequel une teneur de l'acide libre de diclofénac est de 0,5% en masse à 8% en masse sur la base d'une masse totale de la couche adhésive.
